# EUROPEAN PATENT APPLICATION

(11) **EP 1 749 474 A1**
(43) Date of publication of application: **07.02.2007**
(21) Application number: 05254804.7
(22) Date of filing: 01.08.2005
(51) Int. Cl.: A61B 5/00, A61B 5/024

(54) **Life sign detection and real-time reporting module**

(71) Applicant: Mitac Technology Corp., Hsin-Chu, Hsien (TW)
(72) Inventor: Peng, Wen-Hung, Jhudong Township Hsinchu County (TW)
(74) Representative: Dearing-Lambert, Peter Richard

(57) **Abstract**

A life sign detection and real-time reporting module (100) is provided, including a housing case (101) installed with a life sign detector (1), and a signal processing circuit (2) connected to the life sign detector (1) to receive signals and stores the signals in a memory. The signal processing circuit (2) includes a personal data memory (207) for storing personal data of the user, a personal medical record memory (208), and a medical facility database (209). A data transmission interface (3) is connected to the signal processing circuit (2), and a wireless communication module (6) is connected to the data transmission interface (3) for transmitting the data and the signals to a remote base (8). The data transmission interface (3) can be further connected to a global positioning system (GPS) module (7) for receiving GPS signals from a satellite (71), including the current location information, such as longitude, latitude, and altitude.

## Description

### FIELD OF THE INVENTION

The present invention relates to a human body signal detection system and, more particularly, to a life sign detection and real-time reporting module having a life sign detector for receiving the life signals of a user. The received life signals are processed by a signal processing circuit and transmitted by a wireless communication module with the personal medical record to a remote location.

### BACKGROUND OF THE INVENTION

A plurality of detection elements and devices has been developed for detecting human body signals, and various applications are made, such as heart-beat/pulse detector and brain signal detector. In the current bio-technological development, a wide variety of biochips are also devised to accurately detect the changes of ions in the human body and a micro-processing controller can be used to analyze the body condition. Different types of life sign detectors can be used in different applications, such as medical treatment, diagnosis, monitoring, and sports management.

US Patent No. 5,491,474 discloses a conductive structure of a pulse detection device, which uses a conductive rubber and an electrode structure to form a conductive path for the signals. US Patent No. 45102753 discloses a heart-beat monitor designed as a wrist watch structure for easy wearing. US Patent 5,622,180 discloses a heart-beat monitor also designed as a wrist watch. The design includes a wireless heart-beat signal receiver and a heart-beat detection contact electrode for receiving the heart-beat signal through wireless reception or signals transmitted through contact electrode.

In some cases, the design of human body signal detector also integrates the detection of sport signal. US Patent No. 5,891,042 discloses a step counter with wireless pulse detection. The step counter is worn around the waist of the user, and the wireless pulse detector is worn on the chest of the user. The wireless pulse detector detects the heart-beat and sends the signal to the step counter. US Patent No. 6,175,608 discloses a step counter including a wireless step counter, a wireless heart-beat pulse counter, and a wrist watch. The wrist watch includes a receiver for receiving wireless signals from wireless step counter and wireless heart-beat pulse counter.

Although some of the aforementioned patents and technologies provide human body signal integrated with other applications, they can only be used locally. The current technologies and devices cannot provide complete life sign detection, recording, remote transmission and the communication with medical treatment system to provide real time life sign detection and reporting. Therefore, it is imperative to provide a device in such applications.

### SUMMARY OF THE INVENTION

The present invention is made to overcome the aforementioned drawbacks of the conventional technologies. A primary objective of the present invention is to provide a life sign detection and reporting module that can be worn on a specific location of the human body to detect specific a human body signal.

Another objective of the present invention is to provide a life sign detection and reporting module with a remote wireless transmission capability. The life sign detected by the life sign detection and reporting module can be transmitted through a wireless communication module to a pre-defined remote base for data transmission.

Yet another objective of the present invention is to provide a complete life sign detection and real-time reporting module to a medical treatment system. The life sign detection and real-time reporting module can monitor the life signs of the user, and the data, detected signals, personal medical records and medical facility of the choice can be stored in the memory.

Another objective of the present invention is to provide an integrated transmission device between a life sign detection and real-time reporting module and a global position system so that the detected signals can be transmitted to a remote medical facility with the location information, such as longitude, latitude, and altitude.

To achieve the aforementioned goals, the present invention provides a life sign detection and real-time reporting module, comprising a life sign detector, a signal processing circuit connected to the life sign detector for receiving signals and recording a life sign memory. The signal processing circuit also comprises a personal data memory for storing the personal data of the user, a personal medical record memory, and a medical facility memory. A data transmission interface is connected to the signal processing circuit.

A wireless communication module is connected to the data transmission interface for transmitting the detected life sign signals and data in personal data memory to a remote base. The data transmission interface is further connected to a global positioning system (GPS) module for receiving GPS signals from satellites. The GPS signals include current location information of the life sign detection and real-time reporting module, such as longitude, latitude, and altitude.

In comparison with the conventional technologies, the present invention transmits detected life sign through a wireless communication module and reports to a pre-defined remote base, such as medical facility or data center. Furthermore, the life sign detection and real-time reporting module of the present invention monitors the life signs of the user, and the personal data and medical records of the user are stored in the memory. The present invention is applicable in the construction of a complete medical system.

The present invention is integrated with a GPS module so that when a user, such as an elderly or a patent in critical conditions, experiences abnormal health condition, such as low blood pressure and high blood pressure, the life sign detection and real-time reporting module reports the current location information so that the paramedics or ambulance can locate the user through the GPS information transmitted from the present invention.

These and other objects, features, and advantages of the invention will be apparent to those skilled in the art, from a reading of the following brief description of the drawings, the detailed description of the preferred embodiment, and the appended claims.

### BRIEF DESCRIPTION OF THE DRAWINGS

The present invention can be understood in more detail by reading the subsequent detailed description in conjunction with the examples and references made to the accompanying drawings, wherein:
Figure 1 is a schematic view of a life sign detection and real-time reporting module of the present invention;
Figure 2 is a perspective view of a preferred embodiment of the present invention;
Figure 3 is an exploded view of the life sign detection and real-time reporting module disconnected from the connecting device;
Figure 4 is a schematic view of the present invention equipped with a pair of contact electrodes at the bottom of the housing case; and
Figure 5 is a control circuit of signal processing circuit shown in Figure 1.

### DETAILED DESCRIPTION OF THE PREFERRED EMBODIMENTS

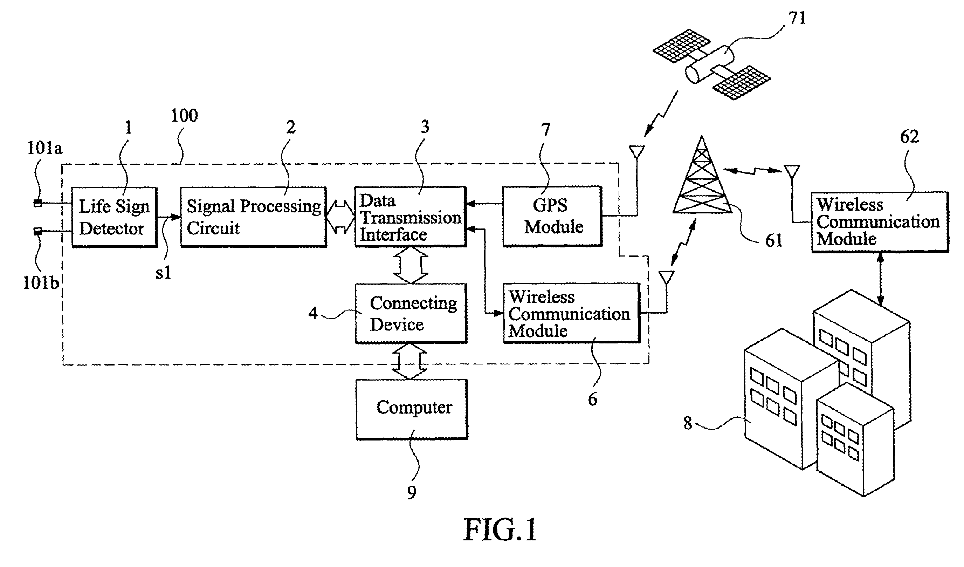

With reference to the drawings and in Particular to Figure 1, a life sign detection and real-time reporting module constructed in accordance with the present invention, generally designated with reference numeral 100, comprises a life sign detector 1, a signal processing circuit 2, a data transmission interface 3, and a connecting device 4. The life sign detection and real-time reporting module 100 can be worn by a user for monitoring human body condition of the user and generates a signal s1. Depending on the type of life sign detector 1, the life sign detection and real-time reporting module 100 can detect different life signs, such as heart-beat pulse signal and brain wave signal. When the life sign detection and real-time reporting module 100 uses for heart-beat monitoring, the life sign detector 1 is connected to a pair of electrodes 101a, 101b.

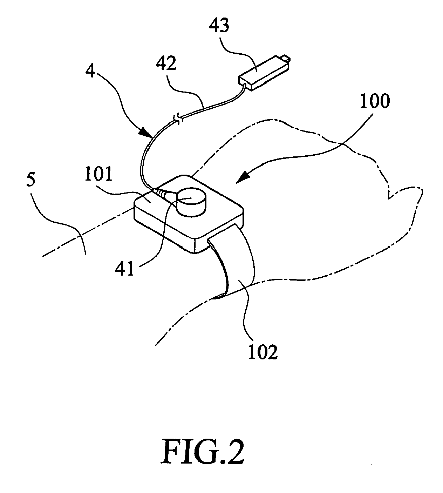

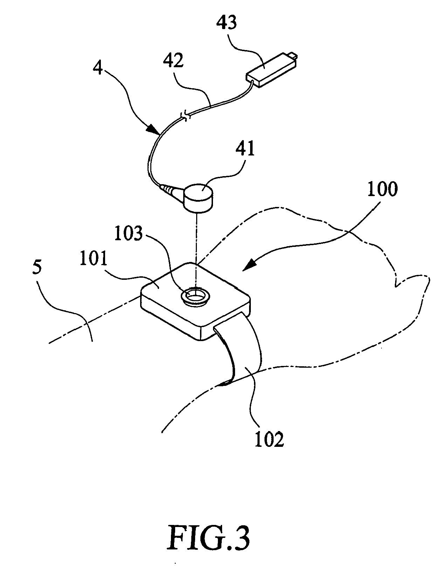

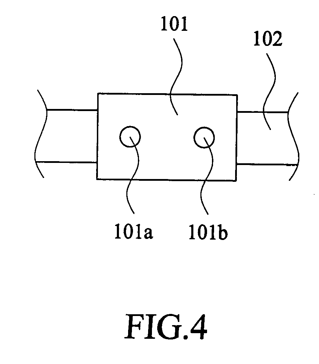

Also referring to Figures 2 and 3, in the embodiment illustrated, the life sign detection and real-time reporting module 100 comprises a housing case 101, which can be made into a shape of wearable item, such as a wrist watch, and strapped to wrist 5 of the user by a strap 102. The housing case 101 comprises a buckle end 103 at the top. As shown in Figure 4, a pair of contact electrodes 101a, 101b is mounted at the location where the bottom of the housing case 101 is in contact with the user's skin.

The connecting device 4 comprises a first plug-in end 41 for being plugged in or buckled up to the buckle end 103 of the housing case 101. When the life sign detection and real-time reporting module 100 is worn on the wrist 5 of the user, the first plug-in end 41 is not plugged in. Therefore, the connecting device 4 can be separated from the buckle end 103 of the housing case 101.

The first plug-in end 41 of the connecting device 4 is connected to a second plug-in end 43 through a signal connecting cable 42. For example, it can be a USB connector for connecting to a USB port of a computer 9 or control device. The connecting device 4 can also be other types of connector, or wireless communication technologies, such as infrared or RF.

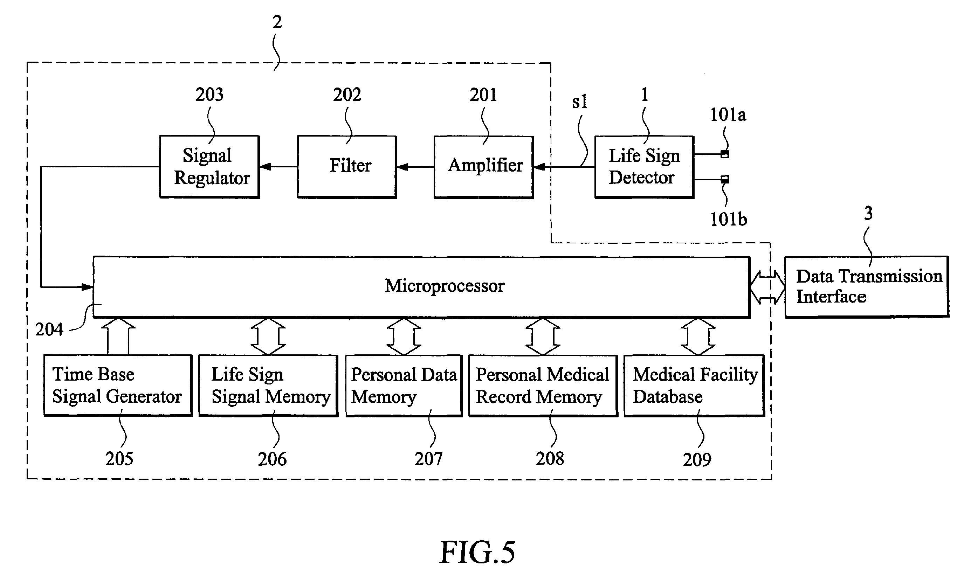

Also referring to Figure 5, which shows a control circuit of the signal processing circuit 2 of Figure 1, the life sign signal s1 detected by the life sign detector 1 is processed by the signal processing circuit 2, including an amplifier 201, a filter 202, and a signal regulator 203. The amplifier 201 amplifies the received signal, the filter 202 filters the noise from the amplified signal, and the signal regulator 203 regulates the waveform of the filtered signal. After processing, the signal s 1 is transmitted to a microprocessor 204.

The microprocessor 204 is connected to a time base signal generator 205 for providing time base signal to the microprocessor 204. The microprocessor 204 is connected to the life sign signal memory 206 for storing the detected signals by the detection circuit 101. When storing the signals, the time field is used for recording the day, month, and year when the signals are recorded.

The microprocessor 204 is also connected to a personal data memory 207 for storing personal data, such as ID, age, gender, height, weight, and medical ID. The personal data can be stored in the computer device 9 through data transmission interface 3 in advance.

The microprocessor 204 is further connected to a personal medical record memory 208 for storing personal medical records. Each time the user goes for medical care, the record is stored in the personal medical record memory 208, such as date, doctor's ID, disease code, and medicine code. The personal medical record can be referred to during the medical diagnosis to save medical test expenses.

The microprocessor 204 is also connected to a medical facility database 209 for storing location and contact information of major or specific medical facilities, such as address, and telephone numbers.

To use the present invention, the user can wear the life sign detection and real-time reporting module 100 on the wrist. The life sign detection and real-time reporting module 100 monitors the life signs specified by the life sign detector, such as heart-beat detector, brainwave activity detector, or various detectors made with bio-chips. A single detector or a plurality of detectors can be used in the application, and all the signals can be transmitted through different channels to the microprocessor 204 for processing to provide a complete view of the human body conditions.

The data transmission interface 3 is connected the computer device 9 through the connecting device 4 so that the signals from the life sign detection and real-time reporting module 100 is sent to the computer device 9 through the connecting device 4 for data analysis. The computer device 9 can be a general purpose personal computer (PC) or a microprocessor-based control device.

The data transmission interface 3 is also connected to a wireless communication module 6, such as commercial GSM, GPRS, or other specific police or medical wireless communication channels. The life sign detection and real-time reporting module uses the wireless communication module 6 to communicate with a wireless communication module 62 of a remote base 8, such as a hospital or a data center, through a wireless communication base station 61 for data uploading and downloading.

In emergency, the life signs of the patient can be transmitted through the wireless communication module 6, the wireless communication base station 61, and the wireless communication module 62 to a remote hospital so that immediate medical care can be performed upon the arrival of the patient.

The data transmission interface 3 is also connected to a GPS module 7 to receive GPS signals from a satellite 71. The GPS signals include the current location information, such as longitude, latitude and altitude of the life sign detection and real-time reporting module 100. When the user, such as an elderly or a patent in critical conditions, experiences abnormal health condition, such as low blood pressure and high blood pressure, the life sign detection and real-time reporting module 100 reports the current location information to the remote base 8 so that the paramedics or ambulance can locate the user through the GPS information transmitted from the life sign detection and real-time reporting module 100.

When in use, the life sign detection and real-time reporting module 100 can also transmit the current location to the remote base 8 and saves the detected life sign signals to the memory in the module for later access, analysis and reference.

While the invention has been described in connection with what is presently considered to the most practical and preferred embodiment, it is to be understood that the invention is not to be limited to the disclosed embodiment, but on the contrary, is intended to cover various modifications and equivalent arrangement included within the spirit and scope of the appended claims.

## Claims

1. A life sign detection and reporting module (100), comprising a housing case (101) wearable to a specific location on a user's body, **characterized in that** a life sign detector (1) arranged inside the housing case (101) for monitoring body conditions of the user and generating a life sign signal (s1), a signal processing circuit (2) comprising a microprocessor (204) connected to the life sign detector (1) for receiving and storing the life sign signal (s1) in a life sign signal memory (206), and a personal data memory (207) for storing personal data, a data transmission interface (3) connected to the signal processing circuit (2), and a wireless transmission module (62) connected to the data transmission interface (3) to transmit received life sign signal (s1) and the personal data stored in the personal data memory (207) to a remote base (8).

2. The module as claimed in Claim 1, **characterized in that** the housing case (101) comprises a buckle end (103) to provide connection to a separable connecting device (4).

3. The module as claimed in Claim 2, **characterized in that** the connecting device (4) comprises:
a first plug-in end (41), connecting to the buckle end (103) of the housing case (101);
a second plug-in end (43), adapted to connect to a computer device (9); and
a signal connecting cable (42), connected to the first plug-in end (41) and the second plug-in end (43).

4. The module as claimed in Claim 1, **characterized in that** the signal processing circuit (2) comprises a personal medical record memory (208) for storing personal medical records of the user.

5. The module as claimed in Claim 1, **characterized in that** the signal processing circuit (2) comprises a medical facility database (209) for storing medical facility information.

6. The module as claimed in Claim 1, **characterized in that** the data transmission interface (3) is connected to a GPS module (7) for receiving GPS signals from a satellite (71), and the GPS signals include current location information, including longitude, latitude and altitude.

7. A life sign detection and reporting module (100), comprising a housing case (101) wearable to a specific location on a user's body, **characterized in that** a life sign detector (1) arranged inside the housing case (101) for monitoring body conditions of the user and generating a life sign signal (s1), a signal processing circuit (2) comprising a microprocessor (204) connected to the life sign detector (1) for receiving and storing the life sign signal (s1) in a life sign signal memory (206), and a personal data memory (207) for storing personal data, a data transmission interface (3) connected to the signal processing circuit (2), a GPS module (7) for receiving GPS signals from a satellite (71) and the GPS signals comprising current location information, including longitude, latitude and altitude of the module (100), and a wireless transmission module (6) connected to the data transmission interface (3) to transmit received life sign signal (s1) and the personal data stored in the personal data memory (207) to a remote base (8).

8. The module as claimed in Claim 7, **characterized in that** the housing case (101) comprises a buckle end (103) to provide connection to a separable connecting device (4).

9. The module as claimed in Claim 8, **characterized in that** the connecting device (4) comprises:
a first plug-in end (41), connecting to the buckle end (103) of the housing case (101);
a second plug-in end (43), adapted to connect to a computer device (9); and
a signal connecting cable (42), connected to the first plug-in end (41) and the second plug-in end (43).

10. The module as claimed in Claim 7, **characterized in that** the signal processing circuit (2) comprises a personal medical record memory (208) for storing personal medical records of the user.

11. The module as claimed in Claim 7, **characterized in that** the signal processing circuit (2) comprises a medical facility database (209) for storing medical facility information.
